# EUROPEAN PATENT SPECIFICATION

(11) **EP 0 695 544 B1**
(45) Date of publication and mention of the grant of the patent: **08.11.2000**
(21) Application number: 95112088.0
(22) Date of filing: 01.08.1995
(51) Int. Cl.: A61K 9/48

(54) **Hard gelatin capsules resistant to denaturation and the production thereof**
Gegen Denaturierung beständige Hartgelatine-Kapseln und Verfahren zu ihrer Herstellung
Capsules en gélatine dure résistantes à la dénaturation et procédé de leur préparation

(30) Priority: 05.08.1994 JP 20432294
(43) Date of publication of application: 07.02.1996
(73) Proprietor: SHIONOGI & CO., LTD., Osaka-shi, Osaka 541-0045 (JP)
(72) Inventor: Sakuma, Satoshi, Otsu-shi, Shiga 520 (JP); Suzuki, Yusuke, Izumi-shi, Osaka 594 (JP); Fujii, Toshiro, Takarazuka-shi, Hyogo 665 (JP); Ogura, Toshihiro, Suita-shi, Osaka 565 (JP); Takagishi, Yasushi, Ashiya-shi, Hyogo 659 (JP)
(74) Representative: VOSSIUS & PARTNER

(56) References cited:
- FR-A- 2 204 401
- FR-A- 2 617 047
- JOURNAL OF PHARMACEUTICAL SCIENCES, vol. 83, no. 7, July 1994 WASHINGTON, DC, US, pages 915-921, G. A. DIGENIS ET AL 'Cross-Linking of Gelatin Capsules and Its Relevance to Their in Vitro-in Vivo Performance'

## Description

This invention relates to hard gelatin capsules resistant to denaturation which are not accompanied by decrease of the solubility or insolubilization time dependently and which release the drug therein steadily. This invention also relates to the production of said capsules.

Hard gelatin capsules which are formulated by packing same contents like drugs in capsule shells made of hard gelatin have widely been used for the improvement of drug handling. In the formulation of acid-susceptible drugs or sustained release preparations, tablets are not suitable because there is much difference in bioavailability among individuals who have taken them. The hard gelatin capsules have usually been utilized to avoid this problem.

For example, it is usual that a granulated drug is coated with an aqueous coating solution of enteric polymer or water-insoluble polymer and packed in hard gelatin capsules in order to minimize the difference in the rate of intestinal drug absorption among persons which depends on the velocity of drug movement in the gastrointestinal tract. Moreover, the formulation by hard gelatin capsules is also utilized to avoid the damage of appearance of drugs, which is sometimes accompanied by the addition of amphiphilic compounds, e.g., Tween (trade name), Span (trade name), polyethylene glycol (PEG), which are added to improve the wettable property of sparingly soluble drugs.

In the above cases, however, the hard gelatin capsules are sometimes denatured during storage under a warmed condition and the drug release is greatly inhibited since the capsules contain aqueous-coating granular drugs or amphiphilic compounds with polyoxyethylene chains.

The reason may be attributable to that PEG, triethyl citrate, etc. used as plasticizer in aqueous coating or the polyoxyethylene chain of the amphiphilic compounds is thermally decomposed to yield peroxidation products such as aldehydes, which act to induce intramolecular or intermolecular bridge formation or polymerization in gelatin.

To solve this problem, for example, organic solvents containing no plasticizer may be used in the coating step. This solution, however, is not desirable because there is a problem of residual solvent since the use of organic solvents is directed to be controlled in view of environmental pollution. In this situation, it is possible to use a plasticizer, e.g., triacetin, glycerol monostearate, which does not produce any peroxide, but they are not suitable because they are inferior in film formation, decomposed with acids to deteriorate acid resistance or drug release time dependently, and emit unpleasant odor, e.g. odor of acetic acid.

It is also known as an alternative method that a protein such as casein, soybean protein, skim milk or collagen is added as an additive to capsules (Japanese Kokai Publication Sho-51-15094). The method, however, is insufficient for inhibiting the generation of peroxides; in order to obtain the desired effect, it is necessary to increase the amount of the additive to be added, which naturally affords a big capsule difficult to engulf. Moreover, the protein added per se is unstable to heat and readily forms the Maillard reaction products with concomitant reducing sugars such as lactose, powdered sugar or refined sugar to deteriorate markedly the appearance of the capsules. This method, accordingly, is insufficient.

FR-A-2 617 047 describes a composition for the preparation of gelatin capsules resistant to tanning by means of the encapsulated products, characterised in that the gelatin contains an ammonium derivative and/or a sulfite derivative, and gelatin capsules prepared with such a composition.

The object of this invention is to provide hard gelatin capsules resistant to denaturation which are not accompanied by decrease of the solubility or insolubilization time dependently and which release the drug therein steadily.

The present invention relates to the subject-matter disclosed in the claims.

The inventors of this invention conducted an extensive study focusing on that peroxidation reaction is one of free radical reactions, and found out that the decrease in hard gelatin capsule solubility and insolubilization is suppressed when a very small amount of a compound with high free radical scavenging activity is added to the capsule content. This invention was accomplished on the basis of these findings.

The gist of this invention is to construct the hard gelatin capsule resistant to denaturation by addition of a free radical scavenger at a rate of 0.01 to 5 weight % to the whole content in said hard gelatin capsule.
Figure 1 and 2 each shows the drug releasing curve for hard gelatin capsules of Example 2.
Figure 3 shows the drug releasing curve for hard gelatin capsules of Example 4.

In the context of this specification, 'free radical scavenger' means anything having free radical scavenging activity (hereinafter referred to as 'RSA'). The mole number of a free radical that can be scavenged by 1 mole of free radical scavenger is referred to as 'RSA value'.

Formulated hard gelatin capsules resistant to denaturation of this invention contain said free radical scavenger and other necessary contents such as drugs and additives in the hard gelatin capsule shells.

The free radical scavenger mentioned above includes anything having a free radical scavenging activity; particularly organic compounds, inorganic compounds and their pharmaceutically acceptable salts having RSA values of not less than 0.01 are preferred. This includes, for example, pharmaceutically acceptable salts of sulfite and hydrogensulfite, as well as cysteine, glutathione, tocopherol, ascorbic acid, thiamine nitrate, riboflavin, β-carotene, acetaminophen, chlorphenilamine maleate, chlorpromazine, pindorol, sesaminol, gossypol, soybean saponine, rosemarinic acid, geraniin, quercetin, glycyrrhyzinic acid, polyphosphoric acid, pyrophosphoric acid, metaphosphoric acid, ferric chloride, etc. and their pharmaceutically acceptable salts. Among them, pharmaceutically acceptable sulfites and hydrogensulfites; and tocopherol, ascorbic acid, polyphosphoric acid, pyrophosphoric acid and their pharmaceutically acceptable salts are preferably used, and moreover, sodium sulfite, tocopherol, pyrophosphoric acid, sodium pyrophosphate and potassium pyrophosphate are particularly preferred.

The amount of said free radical scavenger to be contained is 0.01 to 5 weight % relative to the whole content in the capsules, i.e. the sum of said free radical scavenger and other contents in the capsule. An amount of not more than 0.01 weight % is not sufficient in avoiding the insolubilization of the gelatin capsule shells, and the amount of over 5 weight % is not effective in increasing the dissolving effect and only results in bulkiness of capsules. Particularly preferred, the amount of the free radical scavenger is 0.01 to 1 weight %.

The other contents mentioned above can be anything except those containing aldehyde as a component. Drugs and additives etc. that are generally employed in formulated capsules can be conveniently employed as well.

Said free radical scavenger and said other component used in this invention may be in any form selected from powder, granules, semisolid, solution and the like. Said granules may be coated with an aqueous coating solution or organic solvent.

According to this invention, said free radical scavenger may be selected in accordance with the combination of drugs and additives; the additives mean excipients, preservatives, disintegrators, coloring agents etc. If desired, one or more kinds of said free radical scavengers can be used in combination.

Said hard gelatin capsule shells mean those commonly used in the art of pharmaceuticals, e.g., Gelatin Capsule III, but are not limited thereto.

The hard gelatin capsules resistant to denaturation of this invention may be produced by conventional methods. For example, said free radical scavenger may be packed into the hard gelatin capsule shells after simply being mixed with the powder or granules of the pharmaceutical composition. Occasionally, said free radical scavenger is incorporated into said granules or their coating layer.

The insolubilization of gelatin capsules is attributable to the following phenomenon: PEG used in aqueous coating or the polyethylene chain of an amphipatic compound produces peroxidation products such as aldehydes etc., which react with the amino group of gelatin to form a thin film. According to this invention, the free radical generated by time dependent peroxidation of the capsule content during storage under a warmed condition is trapped by the free radical scavenger so that the peroxidation reaction is prevented. Through this process, the generation of aldehyde is suppressed and as a result the formulation of a thin film on the gelatin capsules and insolubilization are inhibited even though PEG and the like Are used as fillers.

The effect of this invention is as follows.

According to this invention, time dependent decrease in solubility and insolubilization is prevented to provide hard gelatin capsules resistant to denaturation having stable drug releasing properties. EXAMPLES

The following examples further illustrate this invention, however, they do not limit the scope of this invention. All 'parts' in the following examples represent 'weight parts'.

### Measurement of RSA values

Each sample was dissolved or dispersed evenly in 0.1M acetic acid buffer (pH 5.5) or methanol. In a stoppered test tube were placed 2 ml of sample solution, 1 ml of 0.6 mM DPPH · methanol solution, and 2 ml of methanol or 0.1M acetic acid buffer (pH 5.5),and the mixture was agitated at room temperature, then centrifuged; the resulting supernatant was collected and the change of absorbance at 530 nm was measured to give the RSA value.

### Example 1

In a weighing bottle were placed 95 parts of PEG 6000 and 5 parts of free radical scavengers indicated in table 1, on which a basket containing some empty gelatin capsule III was mounted. The capsule shells and the mixture were stored in a tightly closed glass bottle so that they were kept not to contact each other, at 60°C for a week. Each capsule shell was put in an auxiliary tube for the JP12 disintegration test, then the auxiliary tubes were moved into a glass vessel filled up with about 30 ml of the 2nd solution of JP12 (37 °C) carefully, and after the lapse of 5 to 6 minutes, the solubility was observed. The result is shown in Table 1.

**Table 1**

| Free radical scavenger | Solubility |
|---|---|
| none | thin film formed, insoluble |
| ascorbic acid | soluble rapidly |
| riboflavin | soluble rapidly |
| d-α -tocopherol | soluble rapidly |
| thiamine nitrate | soluble rapidly |
| sodium sulfite | soluble rapidly |

According to Example 1, the capsule shells made with PEG 6000 containing no free radical scavenger formed an insoluble thin film; addition of ascorbic acid, riboflavin, tocopherol, thiamine nitrate or sodium sulfite respectively, completely prevented the decrease of the solubility of the capsule shells.

### Example 2

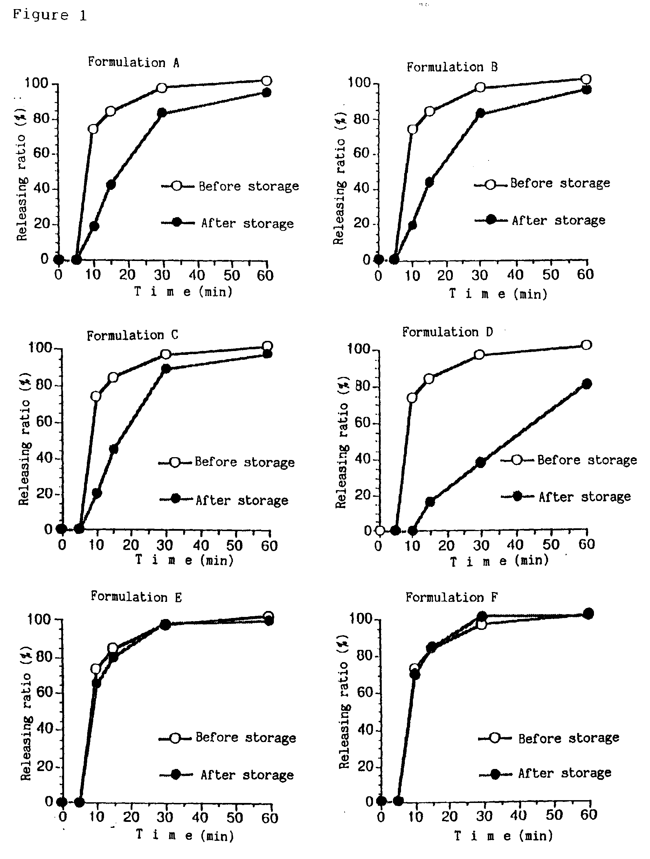

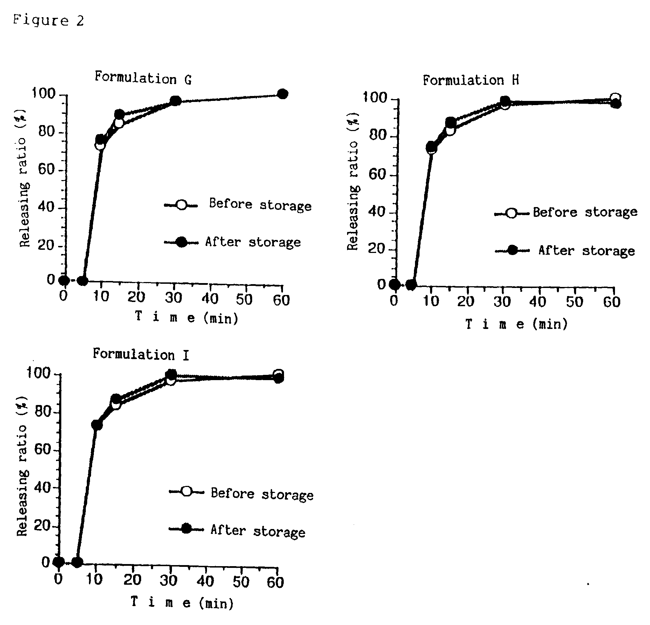

Coated granules (herein after referred to as 'Mixture A') were prepared by mixing 30.0 mg of cristalline cellulose, 10.0 mg of pyridoxine hydrochloride, 54.0 mg of lactose, 5.0 mg of corn starch, 31.5 mg of talc, 0.3 mg of aerosil®, 1.2 mg of magnesium stearate, 7.7 mg of triethyl citrate, 0.5 mg of HPC-L, and 38.3 mg of HPMC-AS, and a variety of compounds indicated in Table 2 were added to Mixture A to give the compositions which were packed into a gelatin capsule III. After having been stored in a tightly closed glass bottle at 60 °C for 5 days, the releasing test was conducted. The releasing test was effected according to the Paddle method of the Japanese Pharmacopoeia (paddle speed: 100 rpm), using 900 ml of the 2nd solution of JP 12 as releasing solution. The results are shown in Figures 1 and 2.

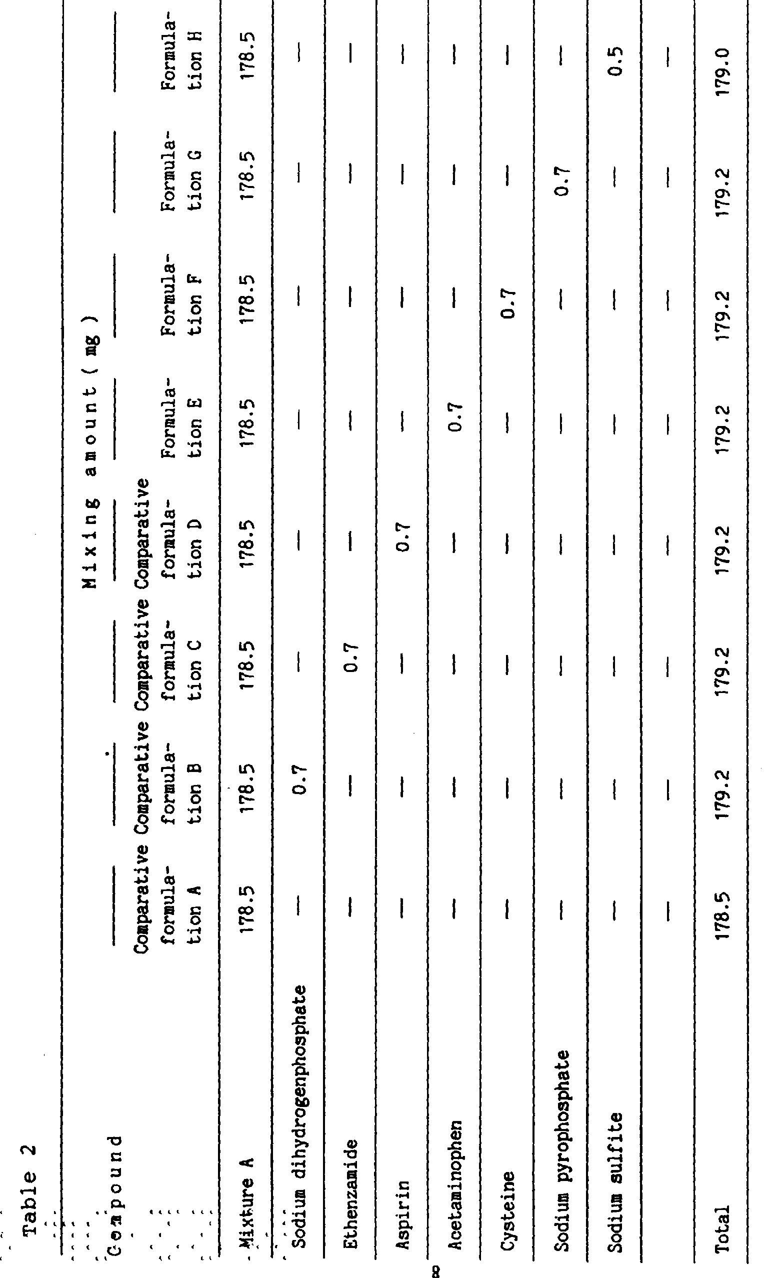

According to Example 2, Comparative formulation A with only Mixture A, and Comparative formulations B, C and D containing sodium hydrogenphosphate, ethenzamide and aspirin, respectively, have no scavenging effect, resulting in insolubilization of the capsules and reducing their drug releasing ability after storage with heat. On the other hand, Formulations E, F, G and H containing acetaminophen, cysteine, sodium pyrophosphate and sodium sulfite, respectively, having great RSA values, did not cause insolubilization of the capsules, and no reduction of the drug releasing ability was observed.

### Example 3

Five parts of each free radical scavenger indicated in Table 3 and 95 parts of PEG 4000 were admixed in a mortar, and packed into gelatin capsule III. After storage in a tightly closed glass bottle at 60°C for a week, the capsule content was removed and solubility of each capsule shell was examined in the same manner as in Example 1. The result is shown in Table 3.

**Table 3**

| Free radical scavenger | Solubility |
|---|---|
| none | thin film formed, insoluble |
| d-α -tocopherol | soluble rapidly |
| polyphosphoric acid | soluble rapidly |
| metaphosphoric acid | soluble rapidly |
| glutathione | soluble rapidly |

According to Example 3, when no free radical scavenger was added, the capsules formed a thin film inducing insolubilization. On the other hand, when the free radical scavenger was added, no decrease in solubility of the capsules was observed after storage under warm conditions.

### Example 4

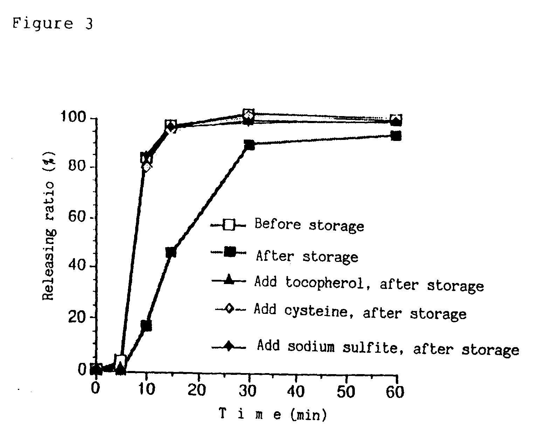

Ten parts of ethenzamide, and 5 parts of each tocopherol, cysteine, or sodium sulfite were dissolved in 50 parts of Tween 80 (trade name) and 35 parts of Span 20 (trade name). The mixture was packed into gelatin capsule shells, stored in a tightly closed glass bottle at 60°C for a week. The releasing test was effected in the same manner as in Example 2. The result is shown in Figure 3.

According to Example 4, while no addition of tocopherol or other free radical scavengers caused insolubilization of the capsules and decrease in drug releasing ability was observed, when added these scavengers, no insolubilization and decrease in drug releasing ability was observed.

## Claims

1. A formulated hard gelatin capsule resistant to hard gelatin capsule shell as a packed component at a rate of 0.01 to 5 weight % for the whole content in the capsule, with the proviso that the capsule does not contain sodium bisulfite, sodium hydrogensulfide or mixtures thereof.

2. The formulated hard gelatin capsule according to claim 1, wherein the content of said free radical scavenger is 0.01 to 1 weight % for the whole content in the capsule.

3. The formulated hard gelatin capsule according to claim 1 or 2, wherein said free radical scavenger is one or more selected from the group consisting of tocopherol, ascorbic acid, polyphosphoric acid, pyrophosphoric acid and the pharmaceutically acceptable salts thereof.

4. The formulated hard gelatin capsule according to claim 3, wherein said free radical scavenger is sodium pyrophosphate and/or potassium pyrophosphate.

5. The formulated hard gelatin capsule according to any one of claims 1 to 4 additionally containing at least one pharmaceutically active compound.

6. A method for producing a formulated hard gelatin capsule resistant to denaturation, wherein a free radical scavenger is packed into the hard gelatin capsule shell at a rate of 0.01 to 5 weight % for the whole content in the capsule, with the proviso that the capsule does not contain sodium bisulfite, sodium hydrogensulfide or mixtures thereof.

7. The method according to claim 6, wherein the content of said free radical scavenger is 0.01 to 1 weight % for the whole content in the capsule.

8. Use of the formulated hard gelatin capsule resistant to denaturation according to any one of claims 1 to 5 as a sustained release preparation.

9. Use of a free radical scavenger to prevent the decrease of solubility of a hard gelatin capsule during storage, with the proviso that the radical scavenger is not sodium bisulfite, sodium hydrogensulfide or mixtures thereof.

10. The formulated hard gelatin capsule according to claim 1 or 2, wherein the contents of the capsule are free from aldehyde as a component.

11. The formulated hard gelatin capsule according to claim 1 or 2, wherein said free radical scavenger is one or more selected from the group consisting of thiamine nitrate, cysteine, glutathione, polyphosphoric acid, and the pharmaceutically acceptable salts thereof.

## Patentansprüche

1. Formulierte Hartgelatine-Kapsel, beständig gegen Denaturierung, welche einen Fänger für freie Radikale als Einschlusskomponente in der Hülle der Hartgelatine-Kapsel in einem Anteil von 0,01 bis 5 Gew.-% bezogen auf den gesamten Inhalt in der Kapsel enthält, mit der Maßgabe, dass die Kapsel Natriumbisulfit, Natriumhydrogensulfid oder Gemische davon nicht enthält.

2. Formulierte Hartgelatine-Kapsel nach Anspruch 1, wobei der Gehalt des Fängers für freie Radikale 0,01 bis 1 Gew.-%, bezogen auf den gesamten Inhalt in der Kapsel, beträgt.

3. Formulierte Hartgelatine-Kapsel nach Anspruch 1 oder 2, wobei es sich bei dem Fänger für freie Radikale um einen oder mehrere handelt, ausgewählt aus Tocopherol, Ascorbinsäure, Polyphosphorsäure, Pyrophosphorsäure und pharmazeutisch verträglichen Salzen derselben.

4. Formulierte Hartgelatine-Kapsel nach Anspruch 3, wobei der Fänger für freie Radikale Natriumpyrophosphat und/oder Kaliumpyrophosphat ist.

5. Formulierte Hartgelatine-Kapsel nach einem der Ansprüche 1 bis 4, die zusätzlich mindestens einen Wirkstoff enthält.

6. Verfahren zur Herstellung einer formulierten Hartgelatine-Kapsel, die gegen Denaturierung beständig ist, wobei ein Fänger für freie Radikale in einem Anteil von 0,01 bis 5 Gew.-%, bezogen auf den gesamten Inhalt in der Kapsel, in der Hülle der Hartgelatine-Kapsel eingeschlossen ist, mit der Maßgabe, dass die Kapsel Natriumbisulfit, Natriumhydrogensulfid oder Gemische davon nicht enthält.

7. Verfahren nach Anspruch 6, wobei der Gehalt des Fängers für freie Radikale 0,01 bis 1 Gew.-% beträgt, bezogen auf den gesamten Inhalt in der Kapsel.

8. Verwendung der gegen Denaturierung beständigen, formulierten Hartgelatine-Kapsel nach einem der Ansprüche 1 bis 5 als Präparat mit Langzeitwirkung.

9. Verwendung eines Fängers für freie Radikale zur Verhinderung der Abnahme der Löslichkeit einer Hartgelatine-Kapsel während der Lagerung, mit der Maßgabe, dass es sich bei dem Radikalfänger nicht um Natriumbisulfit, Natriumhydrogensulfid oder ein Gemisch davon handelt.

10. Formulierte Hartgelatine-Kapsel nach Anspruch 1 oder 2, wobei der Inhalt der Kapsel frei von Aldehyd als Bestandteil ist.

11. Formulierte Hartgelatine-Kapsel nach Anspruch 1 oder 2, wobei es sich bei dem Fänger für freie Radikale um einen oder mehrere handelt, ausgewählt aus Thiaminnitrat, Cystein, Glutathion, Polyphosphorsäure und pharmazeutisch verträglichen Salzen derselben.

## Revendications

1. Capsule de gélatine dure formulée résistante à la dénaturation, qui contient un fixateur radicalaire dans l'enveloppe de la capsule de gélatine dure comme composant de chargement à raison de 0,01 à 5% en poids du contenu total de la capsule, pour autant que la capsule ne contienne pas de bisulfite de sodium, d'hydrogénosulfure de sodium ou leurs mélanges.

2. Capsule de gélatine dure formulée suivant la revendication 1, dans laquelle la teneur en fixateur radicalaire est de 0,01 à 1% en poids du contenu total de la capsule.

3. Capsule de gélatine dure formulée suivant l'une ou l'autre des revendications 1 et 2, dans laquelle le fixateur radicalaire est constitué par un ou plusieurs membres du groupe comprenant le tocophérol, l'acide ascorbique, l'acide polyphosphorique, l'acide pyrophosphorique et leurs sels pharmaceutiquement acceptables.

4. Capsule de gélatine dure formulée suivant la revendication 3, dans laquelle le fixateur radicalaire est du pyrophosphate de sodium et/ou du pyrophosphate de potassium.

5. Capsule de gélatine dure formulée suivant l'une quelconque des revendications 1 à 4, contenant de plus au moins un composé pharmaceutiquement actif.

6. Procédé de production d'une capsule de gélatine dure formulée résistante à la dénaturation, dans lequel un fixateur radicalaire est introduit dans l'enveloppe de la capsule de gélatine dure à raison de 0,01 à 5% en poids du contenu total de la capsule, à la condition que la capsule ne contienne pas de bisulfite de sodium, d'hydrogénosulfure de sodium ou leurs mélanges.

7. Procédé suivant la revendication 6, dans lequel la teneur en fixateur radicalaire est de 0,01 à 1% en poids du contenu total de la capsule.

8. Utilisation de la capsule de gélatine dure formulée résistante à la dénaturation suivant l'une quelconque des revendications 1 à 5, sous la forme d'une préparation à effet retard.

9. Utilisation d'un fixateur radicalaire pour empêcher la diminution de solubilité d'une capsule de gélatine dure en cours de stockage, à la condition que le fixateur radicalaire ne soit pas du bisulfite de sodium, de l'hydrogénosulfure de sodium ou un mélange de ceux-ci.

10. Capsule de gélatine dure formulée suivant l'une ou l'autre des revendications 1 et 2, dans laquelle le contenu de la capsule est exempt d'aldéhyde comme composant.

11. Capsule de gélatine dure formulée suivant l'une ou l'autre des revendications 1 et 2, dans laquelle le fixateur radicalaire est constitué par un ou plusieurs membres du groupe comprenant le nitrate de thiamine, la cystéine, la glutathione, l'acide phosphorique et leurs sels pharmaceutiquement acceptables.
